Europäisches Patentamt

European Patent Office

Office européen des brevets

(11) Publication number: **0 122 888**
**A2**

(12) **EUROPEAN PATENT APPLICATION**

(21) Application number: **84830078.6**

(22) Date of filing: **16.03.84**

(51) Int. Cl.³: **A 61 B 5/04**
**A 61 B 5/00, H 04 M 11/00**

(30) Priority: **18.03.83 IT 937083**

(43) Date of publication of application:
**24.10.84 Bulletin 84/43**

(84) Designated Contracting States:
**AT BE CH DE FR GB LI LU NL SE**

(71) Applicant: **Grassi, Gino**
**Via Imbriani, 21**
**I-50019 Sesto Fiorentino Firenze(IT)**

(72) Inventor: **Grassi, Gino**
**Via Imbriani, 21**
**I-50019 Sesto Fiorentino Firenze(IT)**

(74) Representative: **Lanzoni, Luciano**
**c/o BUGNION S.p.A. Via Farini, 37**
**I-40124 Bologna(IT)**

(54) **Apparatus for telephonically transmitting ECG signals.**

(57) A description is given herein of a simple apparatus that does not have to be connected to any supply source and with which it is possible to utilize certain uncomplicated instruments, currently on the market, for sending in real time through the telephone lines, the twelve standard electrocardiographic leads in respect of the patient, using carrier frequency modulation in the 500 — 3000 Hz range acoustically coupled to the telephone.

FIG1

EP 0 122 888 A2

**0122888**

Apparatus for telephonically transmitting the standard electrocardiographic leads

The apparatus consists, in the example given herein, of a case containing a support on to which are fixed the various components of the circuit and having a compartment in which to house the instrument used.

In modern medicine the diagnostic use of electrocardiograms is becoming evermore widespread and necessary since heart diseases are mainly responsible for sudden death.

Because of the present high degree of complexity of electrocardiograms, the interpretation thereof almost always requires the assistance of a cardiologist whom it may not be possible to have present on every occasion.

One particular situation can be, for example, in the case of myocardial infarction, where rapid diagnosis may save the life of the victim. Another is, for example, in the case of a pacemaker.

Devices have, therefore, been studied that enable suitably equipped Centres to operate a heart clinic that is open 24 hours a day and is reachable simply through the medium of an ordinary telephone.

The said small devices, that can be placed in the charge of the patients, pick up the electrocardiographic signal through electrodes rested, for example, on the chest or attached to the wrists or fingers of the patient by means of straps, and emit an electrocardiographic frequency modulated signal that is generally around 2 kHz. The microphone of the telephone has to be rested on the box in which the instrument is contained, in the region of where the acoustic transducer is position.

When, at the receiving end, there is a demodulator and/or a graphic recorder, the electrocardiogram is recorded in real time and the cardiologist present at the heart clinic is able to give immediately over the same telephone line, the diagnosis and relevant advice.

All the said devices are energized by easily replaceable batteries incorporated therein.

The limitation the devices in question have lies in the fact that they transmit only an unorthodox electrocardiogram that serves solely in cases of obvious arrhythmia, extensive infarction or pacemaker arrhythmia. In other words, they do not transmit the electrocardiogram in the standard leads thereof or, at any rate, in all twelve leads normally required by the cardiologist to enable a diagnosis to be given.

The object of the invention is to make available apparatus that is fully autonomous, contains no active supply (other than the 1 mV calibration signal), and with which it is possible, when the apparatus is easily connected properly to one of the devices briefly mentioned above, to obtain, select, present and transmit through the receiver of an ordinary telephone, the twelve standard leads in respect of any patient, in the required sequence, to the most easily accessible recording and diagnosing centre.

This also makes it possible for the general practitioner to be furnished with an easy, and above all immediate, diagnosis of dangerous cardiac disorders that otherwise are not readily identifiable.

The invention is based on the principle, proved by clinical and laboratory tests, that instruments able to send standard or non-standard leads representing the electric cardiac activity can, with the same fidelity, transmit any one standard lead that the circuit presented is able to select and submit in the appropriate form and sequence.

Furthermore, the apparatus forming the subject of the invention also enables, in the same way, a calibration signal of a known amplitude, generally 1 mV (as required by American Heart Association standards), to be sent and this indispensably completes the electrocardiographic picture.

The apparatus in question is exemplified below in a type of embodiment suitable for the instrument known as the CARDIOTEL, made by Messrs. Instromedix Incorporated of the United States of America. This does not exclude a modification being made, essentially of a mechanical nature, so that the apparatus be able to accept other similar instruments.

In the example given, the apparatus consists of a case (utilized to facilitate handling and carrying) containing a support on to which are fixed the various components of the circuit and having a compartment in which to house the selected instrument, which in the case in question is the CARDIOTEL.

Further details of the apparatus according to the invention will be given below with reference to the following drawings, in which :

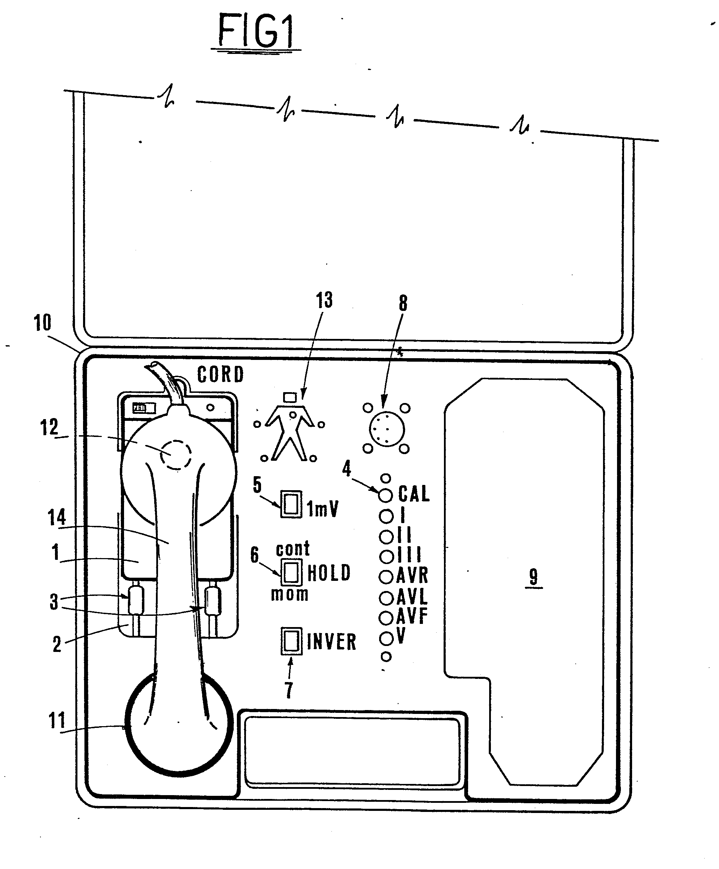

- Figure 1 shows, by way of an example, how the apparatus is laid out inside a case;
- Figure 2 shows the basic electric circuit of the apparatus.

The inside of the case (10) is shaped suitably in such a way as to house and restrain the CARDIOTEL instrument (1) in a compartment (2).

Coming forth out of the said compartment are plugs (3) that connect the CARDIOTEL to the external circuit. A circular cavity (11) faced with elastic material is provided for the telephone receiver (14) to rest in, so positioned that the microphone be placed over the acoustic transducer (12) located in the CARDIOTEL. The indication CORD serves to aid the positioning of the receiver : it is, in fact, at the cord end that the microphone is located. On the inside bottom part of the case are placed the various controls necessary for operating apparatus.

The pushbutton panel (4) selects all twelve standard leads, the position V serving for the precordial V1 - V6 leads obtained by displacing the contact on the chest.

The INVER. switch (7) serves for inverting the direction of the signal, should this be necessary.

The double HOLD switch with central off position (6) serves for preventing the saturation of the amplifier of the CARDIOTEL instrument (1), above all when changing the precordial leads with the electrode detached from the patient.

The noise signal is shortcircuited by the switch (6) in either a lasting fashion (the "cont." position) or in a transitory way (the "mom." position).

A switch (5) (1 mV) serves for giving the calibration signal. This signal is derived from a divider that the switch (5) connects to a long life battery (mercury, lithium etcetera) and it can be sent along, with the CAL. button depressed on the panel (4), or during recording in one of the leads. The foregoing complies with American Heart Association standards.

A printed circuit with the various components is contained inside the case.

Into the connector (8) has to be inserted the patient cable with which five suitably screened wires are carried to the various positioned outlined in the drawing (13) provided in the case.

A compartment (9) serves for containing, for transportation purposes, all the components and accessories needed for the satisfactory operation of the apparatus :  the patient cable, electrodes for limb attachment, the precordial electrode, paste and a standby battery for the instrument used (in the example given herein a CARDIOTEL).

In the embodiment exemplified herein, as means for changing over the leads use has been made of a pushbutton panel (4) but this can well be substituted with a rotary switch.

The pushbutton panel is provided with means for switching the various normal electrocardiographic leads in and out, and these connect to the input of the amplifier of the CARDIOTEL, in accordance with the prescribed standard procedure, the five electrodes that go from the connector (8) to the patient.

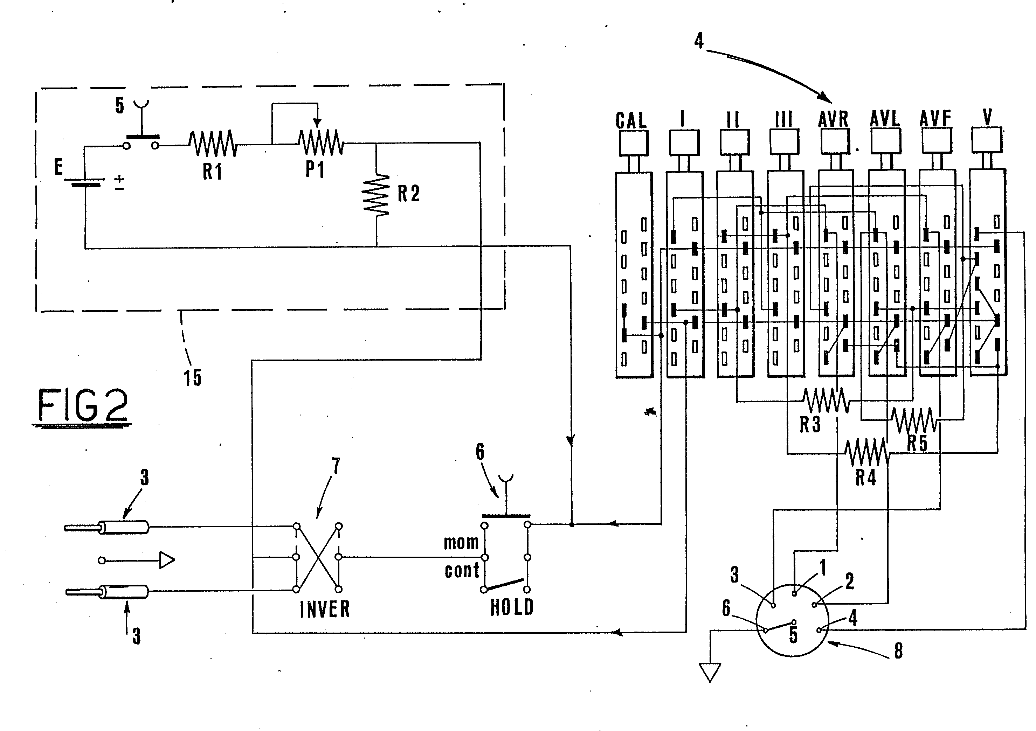

The resistances R3, R4 and R5 (see Figure 2) are all identical and of a value of around 5 kOHM (in the example given herein:  5.1 kOHm).

The calibration circuit is contained in a card (15) that supports the battery E(high stability and long life: mercury, lithium or similar) and the divider R1, P1 and R2 which, when the switch (5) is operated, causes a 1 mV calibration voltage to arrive at the terminals of R2.  R2 has been chosen at a low value (in the example given herein

20 Ohm) in such a way as not to alter the input signal for which the source impedance is very high (5 - 50 kOhm).

The switch (7) is connected to invert the signal at the input should this be upside down on the receiver. The said switch can also be eliminated since it is not essential for the operation of the apparatus.

The switch with a central off position (6) can be placed in a lasting or in a transitory position in order to short-circuit the inputs during the operation of manoeuvring the V1 - V6 leads so as to prevent the pen of the recorder from jolting unduly.

The plugs (3) serve for connecting the apparatus to the chosen instrument (in this case a CARDIOTEL).

The switch on the said instrument sets the whole apparatus in operation, and thus when the instrument is connected to the circuit of the apparatus via the plugs (3), the turning on of the former suffices to render the latter ready for use.

The use of the instrument is exactly like that of an ordinary one pen electrocardiograph.

Claims:

1.  Apparatus for telephonically transmitting the standard electrocardiographic leads, characterized by the fact of comprising a compartment in which to contain a telephonic acoustic transducer, of miniaturized self-powered type, provided with electrical connections (3) that can be linked to the input of the said transducer and terminate at an electric circuit comprising a pushbutton panel (4) with which to select the various standard electrocardiographic leads forthcoming by a patient cable, the said apparatus also comprising a cavity (11) for supporting therein a receiver of a telephone.

2.  Apparatus according to Claim 1, characterized by the fact that the said electric circuit comprises a connector (8), linked to the said selection pushbutton panel, of the five wire type for detachable connection to the said patient cable.

3.  Apparatus according to Claim 1, characterized by the fact that the said electric circuit contains no self powering means.

4.  Apparatus according to Claim 1, characterized by the fact of comprising a self-powered calibration circuit whose output is linked to the electrical connections (3), for generating, when actuated by means of the appropriate pushbutton (5), a constant voltage reference signal.

5.  Apparatus according to Claim 4, characterized by the fact that the said reference signal is a 1 mV signal.

6.  Apparatus according to Claim 1, characterized by the fact that the said electric circuit is provided with a switch (6) able to link, when operated, the said electrical connections to the output of the said pushbutton panel.

7. Apparatus according to Claim 1, characterized by the fact that the said acoustic transducer is of the CARDIOTEL type made by Messrs. Instromedix Incorporated of the United States of America.

# FIG1

FIG2

CAL  I  II  III  AVR  AVL  AVF  V

4

5

E  R1  P1  R2

15

3
3

7
INVER

6
mom
cont
HOLD

R3
R4
R5

3  1  2
6  5  4
8

2/2

0122888